# EUROPÄISCHE PATENTSCHRIFT

(11) **EP 1 368 500 B1**
(45) Veröffentlichungstag und Bekanntmachung des Hinweises auf die Patenterteilung: **27.12.2006**
(21) Anmeldenummer: 02735010.7
(22) Anmeldetag: 18.03.2002
(51) Int. Cl.: C12Q 1/68, C12P 21/02

(54) **VERFAHREN ZUR PRÄPARATIVEN HERSTELLUNG VON LANGEN NUKLEINSÄUREN MITTELS PCR**
METHOD FOR PREPARATIVE PRODUCTION OF LONG NUCLEIC ACIDS BY PCR
PROCEDE DE PREPARATION D'ACIDES NUCLEIQUES LONGS PAR PCR

(30) Priorität: 16.03.2001 DE 10113265
(43) Veröffentlichungstag der Anmeldung: 10.12.2003
(73) Patentinhaber: RiNA Netzwerk RNA-Technologie GmbH, 14195 Berlin (DE)
(72) Erfinder: MERK, Helmut, 14195 Berlin (DE); ERDMANN, Volker, 14163 Berlin (DE); STIEGE, Wolfgang, 14195 Berlin (DE)
(74) Vertreter: Jungblut, Bernhard Jakob
(86) Internationale Anmeldenummer: PCT/DE2002/001047
(87) Internationale Veröffentlichungsnummer: WO 2002/090371

(56) Entgegenhaltungen:
- WO-A-92/07949
- MARTEMYANOV KIRILL A ET AL: "Direct expression of PCR products in a cell-free transcription/translation system: Synthesis of antibacterial peptide cecropin." FEBS LETTERS, Bd. 414, Nr. 2, 1997, Seiten 268-270, XP004366328 ISSN: 0014-5793 in der Anmeldung erwähnt
- GE L ET AL: "SIMULTANEOUS INTRODUCTION OF MULTIPLE MUTATIONS USING OVERLAP EXTENSION PCR" BIOTECHNIQUES, EATON PUBLISHING, NATICK, US, Bd. 22, Nr. 1, 1997, Seite 28,30 XP000676361 ISSN: 0736-6205

### SEQUENCE LISTING

<110> RiNA Netzwerk TNA-Technologien GmbH
<120> Verfahren zur präparativen Herstellung von langen Nukleinsäuren mittel s PCR
<130> RNA/PCT/0201
<140> PCT/DE02/01047
   <141> 2002-03-18
<150> DE 101 13 265
   <151> 2001-03-16
<160> 14
<170> Patent In version 3.1
<210> 1
   <211> 57
   <212> DNA
   <213> artificial
<220>
   <221> primer_bind
   <222> (1)..(57)
   <223>
<400> 1
   taattttgtt taactttaag aaggagatat accatggtgg acgccttcgt gggtacc 57
<210> 2
   <211> 49
   <212> DNA
   <213> artificial
<220>
   <221> primer_bind
   <222> (1) .. (49)
   <223>
<400> 2
   tttaacttta agaaggagat ataccatggt ggacgccttc gtgggtacc 49
<210> 3
   <211> 42
   <212> DNA
   <213> artificial
<220>
   <221> primer_bind
   <222> (1) .. (42)
   <223>
<400> 3
   cgtttagagg ccccaagggg ggtcatgcct gtttctcgta ag 42
<210> 4
   <211> 51
   <212> DNA
   <213> artificial
<220>
   <221> primer_bind
   <222> (1)..(51)
   <223>
<400> 4
   cgaactgcgg gtggctccaa gcgcttgcct gtttctcgta agtacgagtg c 51
<210> 5
   <211> 63
   <212> DNA
   <213> artificial
<220>
   <221> primer_bind
   <222> (1)..(63)
   <223>
<400> 5
   cgtttagagg ccccaagggg gggagtagaa tgttaaggat tagtcatgcc tgtttctcgt 60
   aag 63
<210> 6
   <211> 75
   <212> DNA
   <213> artificial
<220>
   <221> primer_bind
   <222> (1)..(75)
   <223>
<400> 6
   gaaattaata cgactcacta tagggagacc acaacggttt ccctctagaa ataattttgt 60
   ttaactttaa gaagg 75
<210> 7
   <211> 47
   <212> DNA
   <213> artificial
<220>
   <221> primer_bind
   <222> (1)..(47)
   <223>
<400> 7
   gaaattaata cgactcacta tagggtttaa ctttaagaag gagatat 47
<210> 8
   <211> 41
   <212> DNA
   <213> artificial
<220>
   <221> primer_bind
   <222> (1)..(41)
   <223>
<400> 8
   caaaaaaccc ctcaagaccc gtttagaggc cccaaggggg g 41
<210> 9
   <211> 72
   <212> DNA
   <213> artificial
<220>
   <221> primer_bind
   <222> (1)..(72)
   <223>
<400> 9
   caaaaaaccc ctcaagaccc gtttagaggc cccaagggga ttatttttcg aactgcgggt 60
   ggctccaagc gc 72
<210> 10
   <211> 95
   <212> DNA
   <213> artificial
<220>
   <221> primer_bind
   <222> (1)..(95)
   <223>
<400> 10
   caaaaaaccc ctcaagaccc gtttagaggc cccaaggggg ggagtagaat gttaaggatt 60
   agattatttt tcgaactgcg ggtggctcca agcgc 95
<210> 11
   <211> 17
   <212> DNA
   <213> artificial
<220>
   <221> primer_bind
   <222> (1)..(17)
   <223>
<400> 11
   taatacgact cactata 17
<210> 12
   <211> 19
   <212> DNA
   <213> artificial
<220>
   <221> primer_bind
   <222> (1)..(19)
   <223>
<400> 12
   tcacgttgta aaacgacgg 19
<210> 13
   <211> 26
   <212> DNA
   <213> artificial
<220>
   <221> primer_bind
   <222> (1)..(26)
   <223>
<400> 13
   ccggaattct aatacgactc actata 26
<210> 14
   <211> 33
   <212> DNA
   <213> artificial
<220>
   <221> primer_bind
   <222> (1)..(33)
   <223>
<400> 14
   tcgcgacccg ggcaaaaaac ccctcaagac ccg 33

## Beschreibung

### Gebiet der Erfindung

Die Erfindung betrifft ein Verfahren zur präparativen Herstellung von langen Nukleinsäuren mittels PCR sowie verschiedene Verwendungsmöglichkeiten für ein solches Verfahren. Als präparativer Maßstab werden erhaltene Nukleinsäuremengen bezeichnet, die zum unmittelbaren Einsatz in zellfreien Proteinbiosynthesesystemen und/oder in vitro Transskriptionssystemen geeignet sind. Als lange Nukleinsäuren werden solche Nukleinsäuren bezeichnet, die neben einer für ein Protein codierenden Nukleinsäurebasissequenz (beliebiger Länge) weitere Sequenzen, insbesondere regulatorische Sequenzen mit jeweils mehr als 50, sogar mehr als 70, Nucleotiden enthalten. Nukleinsäuren können DNA oder RNA, aber auch PNA sein.

Hintergrund der Erfindung.

Proteine werden für biotechnologische und medizinische Anwendungen in hoher Reinheit, insbesondere aber auch in hoher Menge, i.e. im mg- und g- Maßstab benötigt.
Im Falle von größeren Proteinen ist eine klassische Synthese kaum möglich und jedenfalls unwirtschaftlich.

Eine Möglichkeit der Herstellung von Proteinen in größerem Maßstab ist die gentechnische Herstellung. Hierzu wird klonierte DNA, welche für das gewünschte Protein codiert, als Fremd DNA in Form von Vektoren bzw. Plasmiden in Zellen, insbesondere prokaryontische Zellen eingeschleust. Diese Zellen werden dann kultiviert, wobei die durch die Fremd DNA codierten Proteine exprimiert und gewonnen werden. Zwar lassen sich auf diesem Wege bereits beachtliche Mengen an Protein gewinnen, die insofern bekannten Maßnahmen, insbesondere die Klonierung, sind jedoch aufwendig. Zudem sind die Zellen meist nur transient transfiziert und zudem nur in Ausnahmefällen stabil immortalisiert. Eine kontinuierliche Produktion von Protein erfordert daher einen stetigen Nachschub an frischen Zellen, welche wiederum mittels der vorstehend beschriebenen aufwendigen Maßnahmen hergestellt werden müssen.

Ein weiterer Ansatz ist die sogenannte zellfreie in vitro Proteinbiosynthese. Hierbei werden biologisch aktive Zellextrakte eingesetzt, welche von natürlicherweise vorliegenden zellulären Nukleinsäuren weitgehend befreit sind und welche mit Aminosäuren, energieliefernden Substanzen und zumindest einer Nukleinsäure versetzt werden. Die zugesetzte Nukleinsäure codiert dabei für das herzustellende Protein. Wenn als Nukleinsäure DNA eingesetzt wird, ist die Anwesenheit einer DNA-abhängigen RNA-Polymerase erforderlich. Es kann natürlich auch direkt RNA, mRNA, eingesetzt werden. Auf diesem Wege lassen sich nicht nur solche Proteine in kurzer Zeit und mit vergleichsweise moderatem Aufwand herstellen, die auch gentechnisch herstellbar sind, vielmehr können sogar Proteine hergestellt werden, die beispielsweise zelltoxisch sind und folglich mit den üblichen gentechnischen Zellsystemen überhaupt nicht nennenswert exprimierbar sind. Allerdings ist es notwendig die zugesetzte Nukleinsäure selbst herzustellen, was mittels gentechnischer Methoden wiederum aufwendig ist. Hinzu kommt, daß es oft wünschenwert ist, nicht natürlicherweise mit einer Proteinsequenz verknüpfte regulatorische Sequenzen sowie sonstige Sequenzen, wie Spacer, einzuführen, um die Effizient der Proteinsynthese zu verbessern.

Eine Alternative zur gentechnischen Herstellung von vollständigen, in der zellfreien Proteinbiosynthese einsetzbaren Nukleinsäuren ist die sogenannte Expressions PCR. In diesen Zusammenhängen spielt die effiziente Einführung regulatorischer Sequenzen (sowie anderer, die Translationseffizienz fördernder Sequenzen) in eine herzustellende Nukleinsäure im Rahmen der Amplifikation eine besondere Rolle. Für die Einführung solcher weiterer Sequenzen in eine Ziel-Nukleinsäure sind sehr lange PCR Primer notwendig. Lange Primer sind einerseits in der Herstellung wiederum aufwendig und erhöhen andererseits die Wahrscheinlichkeit der Generierung inhomogener PCR-Produkte.

Stand der Technik.

Aus der Literaturstelle US-A-5,571,690 ist es bekannt, eine Nukleinsäure in präparativem Maßstab mittels PCR herzustellen, wobei die zu amplifizierende Nukleinsäure bereits alle erforderlichen regulatorischen Sequenzen enthält. Bei den insofern bekannten Maßnahmen ist somit keine Einführung anderer, besserer regulatorischer Sequenzen bzw. Ersatz der vorhandenen regulatorischen Sequenzen durch solche andere bessere Sequenzen möglich. Zudem ist die aus der Amplifikation erhaltene Nukleinsäure nicht unmittelbar in der Proteinsynthese einsetzbar. Schließlich kann nicht gezielt eine spezifische Nukleinsäure aus einem Nukleinsäuregemisch amplifiziert werden, bei gleichzeitiger Umwandlung des durch die spezifische Nukleinsäure codierten Zielgens für die Proteinbiosynthese.

Aus der Literaturstelle WO-A-9207949 ist es bekannt, eine Nukleinsäure mit einer für ein Protein codierenden Sequenz sowie einer regulatorischen Sequenz in mehreren Stufen herzustellen und zu amplifizieren. In einem ersten. Schritt wird die für das Protein codierende Sequenz mit einer Standard-PCR amplifiziert. Dabei dient ein stromaufwärts hybridisierender Primer zur Einführung einer Adaptersequenz für eine sogenannte "Overlap-Extension-PCR". In einer parallelen zweiten Stufe wird der Hybridisierungspartner für die Overlap-Extension-PCR hergestellt. Dazu werden zwei teilkomplementäre Primer hybridisiert und aufgefüllt. Das erhaltene Produkt trägt dabei am 3'-Ende die zum 5'-Ende des Amplifikats aus der ersten Stufe homologe Sequenz der Adaptersequenz sowie eine Promotersequenz und Regulationselemente für die zellfreie Proteinbiosynthese. Die dritte Stufe ist schließlich die Overlap und Extension Reaktion. Die Produkte aus den beiden ersten Stufen werden hybridisiert, zum Doppelstrang aufgefüllt und schließlich mit weiteren Primern amplifiziert. Bei dieser abschließenden Amplifikation wird über einen Primer eine zusätzliche Sequenz vor dem Promoter eingebaut, welche eine verbesserte Transskription bewirken soll. In zwei weiteren Stufen erfolgt die Transkription sowie die Translation in einem zellfreien System. Nachteilig ist hierbei, daß insgeamt vier Schritte bis zum Erhalt der gewünschten mRNA notwendig sind. Weiterhin fehlen 3'-Sequenzen, welche für die Proteinbiosynthese in prokaryontischen Systemen wichtig sind. Auch sind keine Affinitätstagsequenzen oder dergleichen, mittels welcher eine Aufreinigung des erhaltenen Proteins erleichtert wird, eingeführt. Aufgrund der Komplexität des Verfahrens sowie dem Fehlen von 3'-Sequenzen für prokaryontische Systeme dürfte das insofern bekannte Verfahren weder für prokaryontische Systeme noch für eine Anwendung auf Nukleinsäuregemische (cDNA oder Genom Bibliotheken) brauchbar sein.

In der Literaturstelle Martemyanov, K.A., et al.; FEBS Lett. 414:268-270 (1997) ist ein Verfahren beschrieben, welches der Literaturstelle WO-A-9207949 ähnelt. Unterschiede bestehen darin, daß eine Sequenzhomologie zwischen dem 5'-Ende des stromaufwärtigen und dem 3'-Ende des stromabwärtigen Primers vorliegt. Dadurch erfolgt eine Multimerisierung und letztendlich wird ein Polyprotein erzeugt, welches dann wiederum in Monomere gespalten wird. Zusätzlich nachteilig bei dieser Variante ist, daß Polyproteine von nur sehr wenigen Proteinen chemisch spaltbar sind. Zudem ist die Ausbeute recht gering.

Aus der Literaturstelle Nakano, H., et al.; Biotechnol. Bioeng. 64:194-199 (1999) ist ein spezieller Proteinbioreaktor für die Expression von PCR-Produkten in Escherichia coli Lysat bekannt. Dabei werden standard PCR-Produkte ohne spezielle Methodiken zu deren Generierung eingesetzt. Das PCR-Produkt wird dabei deutlich schlechter exprimiert als in einem Plasmid.

Alle vorstehenden Literaturstellen betreffen eukaryontische Systeme.

Zum Hintergrund der Erfindung ist noch auf die Literaturstellen Martemyanov Kiril A. et al, FEBS Letters 414(2):268-270 (1997), WO 92/07949 A und Ge L. et al, Biotechniques 22(1):28-30 (1997) zu verweisen.

Technisches Problem der Erfindung.

Der Erfindung liegt das technische Problem zugrunde, ein Verfahren zur Herstellung von langen Nukleinsäuren, insbesondere mit Proteinsequenzen sowie mit ausgewählten regulatorischen Sequenzen, anzugeben, welches mit geringen Aufwand arbeitet, hohe Mengen an Produkt-Nukleinsäuren ergibt, ohne Mehraufwand für prokayontische Systeme geeignet ist und mittels dessen eine Amplifikation von definierten Nukleinsäuren aus Nukleinsäurebibliotheken möglich ist.

Grundzüge der Erfindung.

Zur Lösung dieses technischen Problems lehrt die Erfindung ein Verfahren zur präparativen Herstellung von langen Nukleinsäuren mittels PCR und mit den folgenden Hybridisierungsschritten: a) eine Nukleinsäurenbasissequenz wird am 3' und 5' Ende, bezogen auf doppelsträngige Nukleinsäuren, wobei die Hybridisierung jeweils am 5'-Ende des sense- und antisense-Stranges erfolgt, mit jeweils einem Adapterprimer A und B, welche jeweils eine mit den jeweiligen Enden der Nukleinsäurebasissequenz hybridisierende Teilsequenz aufweisen, hybridisiert, b) das Produkt aus Stufe a) wird am 3' und 5' Ende, nämlich den außenliegenden freien Enden der Adapterprimer A und B, mit jeweils einem Verlängerungsprimer C und D, welcher eine Verlängerungssequenz enthält und welche jeweils eine mit den jeweiligen Enden der Adapterprimer A und B hybridisierbare Teilsequenz aufweisen, hybridisiert, wobei aus der Nukleinsäurebasissequenz eine an dem 3' und dem 5' Ende der Nukleinsäurebasissequenz um die Verlängerungssequenzen verlängerte und amplifizierte Nukleinsäuresequenz gebildet wird.

Als Nukleinsäurebasissequenz ist eine Sequenz bezeichnet, die für ein Protein codiert. Dabei kann es sich insbesondere um ein Gen handeln, aber auch um Sequenzen aus intronlosen Genomen. Bei den Verlängerungssequenzen kann es sich insbesondere um Sequenzen handeln, die eine regulatorische Sequenz umfassen und/oder um Sequenzen, welche eine ribosomale Bindungssequenz enthalten. Die Adapterprimer sind vergleichsweise kurz. Eine Teil einer Adaptersequenz ist spezifisch für die Nukleinsäurebasissequenz, ein weiterer Teil ist konstant und hybridisiert jeweils einer Varlängerungssequenz.

Hieraus folgt, daß für unterschiedliche Nukleinsäurebasissequenzen nicht jeweils "passende" lange Verlängerungssequenzen eingesetzt werden müssen. Vielmehr müssen lediglich die vergleichsweise kurzen Adapterprimer auf eine definierte Nukleinbasissequenz abgestimmt werden, während die Verlängerungssequenzen gleichsam universell sein können, i.e. für verschiedene Nukleinsäurebasissequenzen sind stets die gleichen bzw. einige wenige ausgewählte Verlängerungsequenzen einsetzbar. Somit können die relativ aufwendig herzustellenden Verlängerungsequenzen einer breiten Nutzung zugeführt werden und für eine spezifische Nukleinsäurebasissequenz müssen lediglich die Adaptersequenzen hergestellt werden. Dies ist jedoch wenig aufwendig, da die Adaptersequenzen vergleichsweise kurz sein können.

Dies erlaubt es beispielsweise, sowohl eine regulatorischen Sequenz als auch eine ribosomalen Bindungssequenz, jeweils über einen der Verlängerungsprimer, an eine Nukleinsäurebasissequenz anzufügen, und zwar sogar in einem PCR Schritt. So kann eine Nukleinsäure erhalten werden, die eine besonders hohe Transskriptions- und/oder Translationseffizienz in einem prokaryontischen System der zellfreien Proteinbiosynthese ergibt.

Ein besonderer Vorteil des erfindungsgemäßen Verfahrens ist, daß es sich um ein allgemein anwendbares Verfahren für beliebige codierende Sequenzen handelt.

Die Hybridisierung mit einem Primer am 3' und 5' Ende bezieht sich insbesondere auf doppelsträngige Nukleinsäuren, wobei die Hybridisierung des Primers jeweils am 5'-Ende des sense- und antisense-Stranges erfolgt. Auf den Einzelstrang bezogen findet die Hybridisierung mit den verschiedenen vorstehend und folgend beschriebenen Primern am jeweiligen 5'-Ende statt.

Ausführungsformen der Erfindung.

Von selbstständiger Bedeutung ist eine Weiterbildung der Erfindung, wobei das Produkt aus der Stufe b) in einer Stufe c) am 3' Ende und am 5' Ende mit jeweils einem Amplifikationsprimer hybridisiert werden kann, wobei eine amplifizierte Nukleinsäureendsequenz gebildet wird. Auch die Amplifikationsprimer sind einerseits vergleichsweise kurz und universell einsetzbar und folglich leicht verfügbar. Mittels der Amplifikationsprimer können zudem weitere (kürzere) Sequenzen an den Enden angefügt werden, welche die Translationseffizienz weiter erhöhen. Auch sind mittels der kurzen Amplifikationsprimer Variationen und Modifikationen an den Enden der Nukleinsäuren mit wenig Aufwand einführbar. Dies ist insbesondere deshalb von Vorteil, da damit für Variationen und Modifikationen keine unterschiedlichen Verlängerungsprimer hergestellt zu werden brauchen, was wiederum in störendem Maße aufwendig wäre.

Ein Beispiel für eine Variation bzw. Modifikation ist der Einbau eines Biotinrestes, am 5'-Ende des Amplifikationsprimers gekoppelt. Hierdurch wird nach Inkubation der Nukleinsäureendsequenz mit beispielsweise Biotin-bindendem Streptavidin eine gegen Exonuklease-Abbau stabilisierte Nukleinsäureendsequenz erhalten, welche eine Erhöhung der Halbwertszeit in einem in vitro Proteinbiosynthesesystem gegenüber einer nicht stabilisierten Nukleinsäureendsequenz um ein Vielfaches, typischerweise mehr als 5-fach, beispielsweise von ca. 15 min. auf ca. 2 h, aufweist. Es werden Stabilitäten erreicht, welche mit jenen von zirkulären Plasmiden vergleichbar sind und diese insofern gleichwertig ersetzen können. Eine Alternative ist eine Stabilisierung mittels Digoxygenin, welches Anti-Digoxygenin-Antikörper bindet. Die Stabilisierende Gruppe kann insbesondere an beiden Enden der Nukleinsäureendsequenz eingerichtet sein. Ein anderes beispiel ist eine Modifikation mit einem Affinitytag bzw. einer hierfür codierenden Sequenz oder eine Ankergruppe bzw. einer hierfür codierenden Sequenz. Eine Ankergruppe erlaubt eine Immobilisierung durch Bindung der Ankergruppe an eine Festkörperoberfläche mit gematchten Bindungssites. Die Ankergruppe kann an der Nukleinsäure selbst angebracht sein, es kann aber auch eine hierfür codierende Sequenz vorgesehen sein.

Die Adapterprimer enthalten typischerweise < 70, insbesondere 20 - 60, Nucleotide. Die Verlängerungsprimer enthalten typischerweise ≥ 70, auch 90 und mehr, Nucleotide. Die Amplifikationsprimer wiederum enthalten typischerweise < 70, meist < 30, Nukleotide, typischerweise > 9 Nukleotide. Lediglich die Adapterprimer müssen an eine definierte Nukleinsäurebasissequenz spezifisch angepaßt werden, was im Lichte der relativ kurzen Sequenzen mit wenig Aufwand verbunden ist.

Vorteilhafterweise werden die Schritte a), b) und optional der Schritt c) in einer PCR Lösung enthaltend die Nukleinsäurebasissequenz, die Adapterprimer, die Verlängerungsprimer und optional die Amplifikationsprimer durchgeführt. Es handelt sich dann um eine Einstufen PCR mit insgesamt 6 Primern, zwei Adaptersquenzen, zwei Verlängerungssequenzen und zwei Amplifikationssequenzen. Dabei reicht es, die Adapterprimer und Verlängerungsprimer in geringen Konzentrationen einzusetzen und insofern auch nur eine geringe Menge an Zwischenprodukt zu erzeugen. Das Zwischenprodukt braucht zudem nicht homogen vorliegen, wodurch aufwendige Optimierungen entfallen können. Aufgrund der Kürze der Amplifikationsprimer sind auch bei der Amplifikation auf die hohe Menge an Nukleinsäureendsequenz keine Optimierungen erforderlich.

Alternativ zu der vorstehenden Ausführungsform ist von selbstständiger Bedeutung eine Variante, die mit zwei PCR Stufen arbeitet. Dabei werden die Schritte a) und b) in einer Verfahrenstufe A) in einer Vor-PCR Lösung enthaltend die Nukleinsäurebasissequenz, die Adapterprimer und die Verlängerungsprimer für eine definierte erste Zyklenzahl durchgeführt und wird der Schritt c) in einer Verfahrenstufe B) in einer Haupt-PCR Lösung enthaltend das PCR Produkt aus der Stufe A) und die Amplifikationsprimer für eine definierte zweite Zyklenzahl durchgeführt. Dabei kann die Stufe A) in einem Reaktionsvolumen durchgeführt werden, welches 1/2 bis 1/10 des Reaktionsvolumens der Stufe B) beträgt. In der Stufe A) entsteht dann, bedingt durch das geringere Volumen eine höhere Konzentration an Zwischenprodukt bzw. es kann deutlich weniger Nukleinsäurebasissequenz eingesetzt werden. Mit der Verdünnung mittels PCR-Ansatzvolumen beim Übergang von der Stufe A) zur Stufe B) werden wiederum die Adapterprimer sowie die Verlängerungsprimer stark verdünnt mit der Folge einer Erhöhung der Wahrscheinlichkeit des Einbaus von Variationen und/oder Modifikationen in die Nukleinsäureendsequenzen über die Amplifikationsprimer.

Im Einzelnen kann in der ersten vorstehenden Alternative so verfahren werden, daß die PCR in einem Reaktionsvolumen von 10 bis 100 µl, vorzugsweise 20 bis 40 µl, mit 0,01 bis 100 pg, vorzugsweise 1 bis 50 pg, Nukleinsäurebasissequenz, 0,05 bis 10 µM, vorzugsweise 0,1 bis 5 µM, Adapterprimer und 0,005 bis 0,5 µM, vorzugsweise 0,001 bis 0,1 µM, Verlängerungsprimer durchgeführt wird, wobei nach einer definierten Anfangszyklenzahl 0,01 bis 10 µM, vorzugsweise 0,1 bis 10 µM, Amplifikationsprimer zugegeben werden, und wobei mittels einer definierte Anzahl anschließender Zyklen die amplifizierte Nukleinsäureendsequenz hergestellt wird. In der zweiten vorstehenden Alternative empfehlen sich die folgenden Reaktionsbedingungen: Stufe A): Reaktionsvolumen < 10 µl; 0,001 bis 5 pg, vorzugsweise 0,01 bis 1 pg, Nukleinsäurebasissequenz; 0,05 bis 10 µM, vorzugsweise 0,1 bis 5 µM, Adapterprimer und 0,05 bis 10 µM, vorzugsweise 0,1 bis 5 µM, Verlängerungsprimer; erste Zyklenzahl 10 bis 30, vorzugsweise 15 bis 25, Stufe B): Reaktionsvolumen 10 bis 100 µl, vorzugsweise 15 bis 50 µl, erhalten durch Ergänzung der Lösung aus Stufe A) mit PCR-Ansatzlösung; 0,01 bis 10 µM, vorzugsweise 0,1 bis 5 µM, Amplifikationsprimer; zweite Zyklenzahl 15 bis 50, vorzugsweise 20 bis 40.

Die Erfindung lehrt weiterhin die Verwendung eines erfindungsgemäßen Verfahrens zur Herstellung von Nukleinsäuren für die zellfreie in vitro Proteinbiosynthese, insbesondere in prokaryontischen Systemen, vorzugsweise in einem Translationsystem aus Escheria coli D10.

Ein erfindungsgemäßes Verfahren ist vorteilhafterweise einsetzbar zur selektiven Amplifikation einer definierten Nukleinsäurebasissequenz aus einer Nukleinsäurebibliothek. Dies ermöglicht eine Charakterisierung von Gensequenzen, wobei die Gensequenz als Nukleinsäurenbasissequenz eingesetzt wird und wobei das erhaltene Protein hinsichtlich Struktur und/oder Funktion analysiert wird. Der Hintergund dieses Aspekts der Erfindung ist, daß für viele Gene zwar die Sequenzen bekannt sind, nicht jedoch die Struktur und Funktion des dadurch codierten Proteins. Somit können Elemente einer Genbibliothek, zu welchen lediglich die Sequenz als solche bekannt ist, auf ihre Funktion in einem Organismus untersucht werden. Die Untersuchung der Struktur und Funktion des erhaltenen Proteins folgt dabei üblichen Arbeitsmethoden der Biochemie.

Mittels des erfindungsgemäßen Verfahrens lassen sich Nukleinsäuren gewinnen, welche eine für ein Protein codierenden Nukleinsäurebasissequenz und eine ribosomale Bindungssequenz sowie optional eine oder mehrere Sequenzen aus der Gruppe bestehend aus "Promotorsequenz, Transskriptionsterminatorsequenz, Expressionsenhancersequenz, Stabilisierungssequenz und Affinitytagsequenz" enthalten. Eine Affinitytagsequenz codiert für eine Struktur, welche eine hohe Affinität für (meist immobilisierte) Bindungsstellen in Trennsystemen zur Aufreinigung aufweist. Dadurch ist eine leichte und hochaffine Abtrennung von Proteinen, welche das Affinitytag nicht enthalten, möglich. Ein Beispiel hierfür ist Strep-tag II, eine Peptidstruktur aus 8 Aminosäureresten mit Affinität zu StrepTactin. Eine Stabilisierungssequenz codiert für eine Struktur, welche entweder selbst, oder nach Bindung mit einem für die Struktur spezifischen Bindungsmolekül, eine Stabilisierung gegen Degradation, insbesondere durch Nukleasen, bewirkt. Eine Stabilisierung einer Nukleinsäure(end)sequenz kann auch dadurch erfolgen, daß an einem Ende, vorzugsweise an beiden Enden, eine Biotingruppe eingebaut ist, welche mit Streptavidin umgesetzt sein kann. Dieser Enbau kann durch Einsatz von Biotin tragenden Primern, insbesondere Amplifikationsprimern, erfolgen. Eine Expressionsenhancersequenz steigert die Translationseffizienz gegenüber einer Nukleinsäure ohne Expressionsenhancersequenz. Dabei kann es sich beispielsweise um (nicht translatierte) Spacer handeln. Eine Transkriptionsterminatorsequenz terminiert die RNA-Synthese. Ein Beispiel hierfür ist der T7 Phage Gen 10 Transskriptionsterminator. Transskriptionsterminatorsequenzen können auch gegen Degradation durch 3' Exonukleasen stabilisieren. Vorteilhafte relative Anordnungen der vorstehenden Sequenzelemente zueinander lassen sich aus den folgenden Ausführungsbeispielen verallgemeinern.

Im Folgenden wird die Erfindung anhand von lediglich bevorzugte Ausführungsformen darstellenden Beispielen näher erläutert.

### Methoden:

### PCR:

Die PCR wurde in einem in den Beispielen quantifizierten Reaktionsvolumen mit 10 mM Tris-HCl (pH 8,85 bei 20°C), 25 mM KCl, 5 mM (NH₄)₂SO₄, 2 mM MgSO₄, 0,25 mM jeder dNTP, 3 U Pwo DNA Polymerase (Roche) sowie der in den Beispielen angegebenen Menge Nukleinsäurebasissequenz durchgeführt. Die Zyklen wurden für 0,5 min. bei 94°C, 1 min. bei 55°C und 1 min. bei 72°C durchgeführt.

### In vitro Expression:

In vitro Experimente wurden gemäß der Literaturstelle Zubay, G.; Annu. Rev. Genet. 7:267-287 (1973) mit den folgenden Modifikationen durchgeführt. Das Escherichia coli S-30 Lysat wurde mit 750 U/ml T7 Phagen RNA Polymerase (Stratagene) und 300 µM [¹⁴C]Leu (15 dpm/pmol, Amersham) supplementiert. PCR Produkte und Kontrollplasmide wurden in Konzentrationen von 1 nM bis 15 nM eingesetzt. Die Reaktionen wurden bei 37°C durchgeführt, wobei der Verlauf dadurch verfolgt wurde, daß zu subsequenten Zeitpunkten 5 µl Aliquoten der Reaktionsmischung entnommen und der Einbau von [¹⁴C]Leu mittels TCA Präzipitation abgeschätzt wurde. Weitere 10 µl Aliquoten wurden zwecks Analyse des synthetisierten Proteins mittels SDS-PAGE, gefolgt von einer Autoradiographie in einem Phosphoimager-System (Molecular Dynamics) entnommen.

### Plasmid Konstruktion:

Ein high copy Derivat des Plasmids pET BH-FABP (Specht, B. et al.; J. Biotechnol. 33:259-269 (1994)), welches für bovine heart fatty acid bindung protein codiert wurde konstruiert, pHMFA genannt. Ein Fragment von pET BH-FABP wurde durch Verdau mit den Endonukleasen SphI und EcoRI erzeugt und in den Vektor pUC18 insertiert. Bezüglich der Sequenzen, welche für die Synthese von H-FABP relevant sind, ist das Plasmid pHMFA identisch mit dem Originalplasmid. Es sei angemerkt, daß das linearisierte Plasmid sich nicht besser als das circuläre,Plasmid verhält.

Konstruktion von Nukleinsäuren mit verschiedenen Sequenzbereichen upstream des Promotors:
Das Plasmid pHMFA diente als Matrize für die Konstruktion von Nukleinsäuren mit verschiedenen Sequenzbereichen upstream des Promotors. Die Konstrukte (siehe Beispiele) FA1, FA2 und FA4 mit 0, 5 und 249 Basenpaaren upstream des Promotors wurden mit den Primern P1, C1 und P2 sowie mit dem downstream Primer P3 generiert. Das Konstrukt FA3 mit einem Sequenzbereich von 15 Basenpaaren upstream des Promotors wurde durch Verdau von FA4 mit der Endonuklease Bgl II erhalten. Das Kontrollplasmid pHMFA(EcoRV) mit einem Sequenzbereich von 3040 Basenpaaren wurde durch Verdau des Plasmids mit EcoRV erhalten. Alle Produkte wurden gereinigt durch Agarose Gel Elektrophorese, gefolgt von Gel Extraktion mittels des "High Pure PCR Product Purification Kit".

### Affinitätsreinigung:

Die Reinigung des fatty acid binding protein enthaltend Strep-tag II (Voss, S. et al.; Protein Eng. 10:975-962 (1997)) wurde mittels Affinitätschromatographie gemäß Herstellervorschrift (IBA Göttingen, Deutschland) durchgeführt mit der Abweichung eines reduzierten Volumens der Affinitätssäule (200 µl). Die Reaktionsmischung der gekoppelten Transkription/Translation wurde kurz zentrifugiert und dann auf der Säule aufgetragen. Isolierte Fraktionen wurden durch TCA Präzipitation und Autoradiographie nach SDS-PAGE (s.o.) analysiert.

### H-FABP Aktivitätsassay:

Die vollständige Reaktionsmischung mit in vitro synthetisiertem H-FABP wurde auf die Aktivität der Bindung von Oleinsäure untersucht. Verschiedene Volumina (0 - 30 µl) wurden mit Reaktionsmischung ohne H-FABP auf 30 µl aufgefüllt und mit Translationpuffer (50 mM HEPES pH 7, 6, 70 mM KOAc, 30 mM NH₄Cl, 10 mM MgCl₂, 0, 1 mM EDTA, 0,002 % NaN₃) auf ein Endvolumen von 120 µl verdünnt. Nach Zugabe von 2 µl 5 mM [9,10(n)-³H] Oleinsäure (Amersham) mit einer spezifischen Aktivität von 1000 dpm/pmol wurden die Proben für eine Stunde bei 37°C inkubiert. 50 µl der Proben wurden zur Entfernung ungebundener Oleinsäure mittels Gelfiltration (Micro Bio Spin Chromatographie Columns; Bio-Rad) eingesetzt. Die ³H Radioaktivität der eluierten Fraktionen wurde mittels eines Scintillationszählers gemessen.

### Analyse der Stabilität der Nukleinsäuren:

Radioaktiv markierte Nukleinsäuren wurden gemäß den vorstehenden Bedingungen synthetisiert, jedoch in Gegenwart von 0,167 µCi/µl [α-³⁵S] dCTP. Die markierten Nukleinsäuren wurden in einer gekoppelten Transskription/Translation, Reaktionsvolumen 400 µl, eingesetzt. 30 µl Aliquoten wurden an aufeinanderfolgenden Zeitpunkten entnommen. Nach der Zugabe von 15 µg Ribonuklease A (DNAse-frei, Roche) wurden diese für 15 min. bei 37°C inkubiert. Eine weitere Inkubation für 30 min. bei 37°C wurde nach Zugabe von 0,5 % SDS, 20 mM EDTA und 500 µg/ml Proteinase K (Gibco BRL) in einem Gesamtreaktionsvolumen von 60 µl durchgeführt. Verbleibende PCR Produkte wurden weiter gereinigt mittels Ethanolfällung und wurden danach einer denaturierenden Elektrophorese (5,3% Polyacrylamid, 7 M Harnstoff, o,1 % SDS, TBE) unterzogen. Das getrocknete Gel wurde zur Quantifizierung der Radioaktivität durch ein Phosphoimager System (Molecular Dynamics) laufen gelassen.

### Sequenzen:

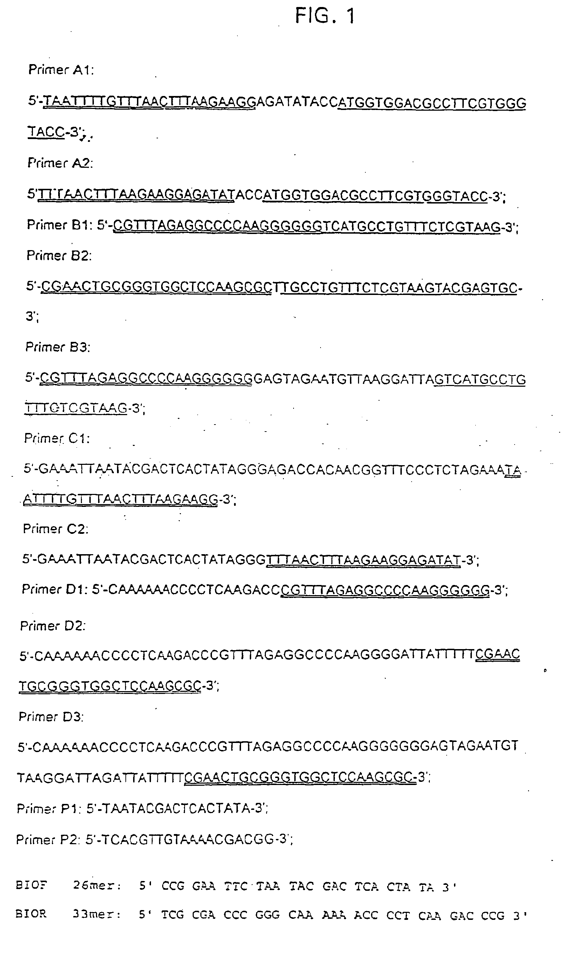

Eingesetzte Primersequenzen sind in der Figur 1 dargestellt.

### Beispiel 1: PCR mit vier Primern

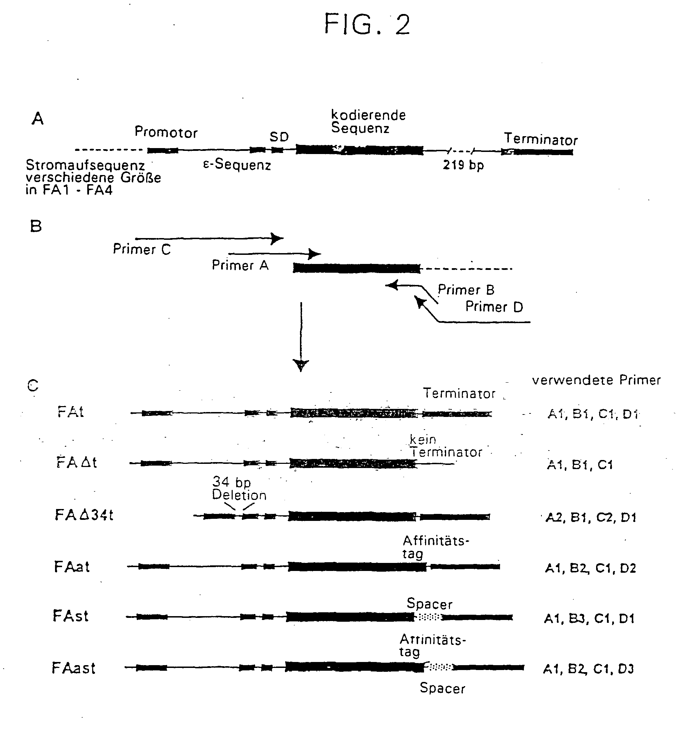

In der Figur 2 ist eine schematische Darstellung einer erfindungsgemäßen Einstufen-PCR mit vier Primern dargestellt. Mittig erkennt man zunächst die für ein Protein codierende Nukleinsäurebasissequenz, welche die vollständige Codierungssequenz für H-FABP (homogeneous and functionally active fatty acid binding protein from bovine heart), erhalten als ein 548 bp Restriktionsfragment aus pHMFA durch Verdau mittels der Endonukleasen Ncol und BamHI, umfaßt (sowie eine 150 bp Sequenz am 3'-Ende, welche weder translatiert wird, noch zu einem Adapterprimer oder Verlängerungsprimer complementär ist). Hieran werden die beiden Adapterprimer A und B hybridisiert, welche mit den Enden der Nukleinsäurebasissequenz homologe Enden umfassen. Der Adapterprimer A enthält weiterhin eine ribosomale Bindungssequenz. An die außenliegenden Enden der Adapterprimer A und B werden die Verlängerungsprimer C und D hybridisiert. Der Verlängerungprimer C umfaßt die T7 Gen 10 Leadersequenz einschließlich des T7 Transskriptionspromotors sowie optional upstream eine Sequenz von beispielsweise 5 Nukleotiden. Der Verlängerungsprimer D umfaßt die T7 Gen 10 Terminatorsequenz.

### Beispiel 2: Effizienz der H-FABP Synthese in Abhängigkeit des Sequenzbereiches upstream des Promotors.

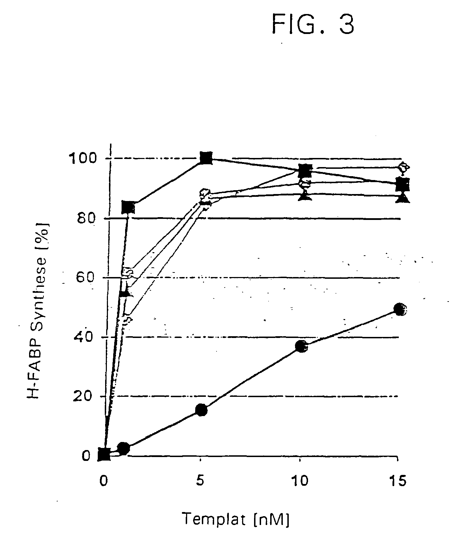

Vier PCR Produkte (FA1 bis FA4) mit verschiedenen Sequenzbereichen upstream des Promotors (0, 5, 15, 250 Basenpaare) und das linearisierte Kontrollplasmid pHMFA(EcoRV) mit 3040 bp upstream des Promotors wurden in verschiedenen Konzentrationen (1, 5, 10 und 15 mM) auf in vitro Transskription/Translation untersucht. Die Figur 3 zeigt, daß alle Sequenzbereiche, außer 0 (untere Kurve), eine Erhöhung der Proteinsynthese bewirken. Bereits 5 Basenpaare reichen aus.

### Beispiel 3: Verbesserung der H-FABP Synthese durch Phage T7 Gen 10 Transskriptionsterminator/5' leader Sequenz Phage T7 Gen 10.

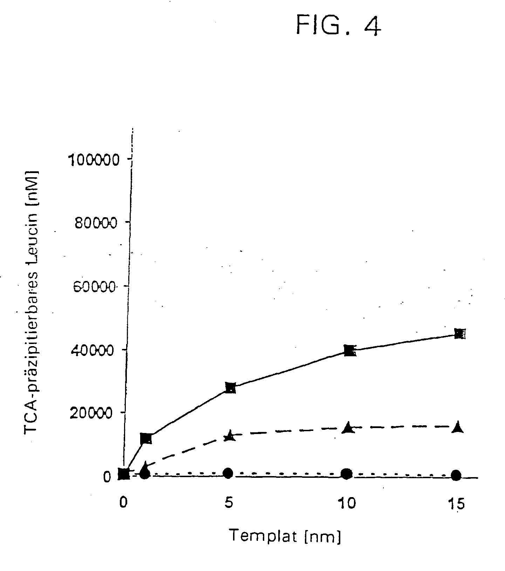

In der Figur 4 erkennt man, daß durch den Phage T7 Gen 10 Transskriptionsterminator die Synthese um zumindest einen Faktor 2, 8 verbessert werden kann. Die Dreiecke stehen für FAΔt und die Quadrate für FAt (siehe auch Fig. 2).

Weiterhin erkennt man in der Figur 4, daß eine Deletion von 34 bp zwischen dem Transskriptionsanfang und der epsilon-Sequenz (Olins, P.O. et al.; Escherichia coli. J. Biol. Chem. 264:16973-16976 (1989) zu einer Unterdrückung einer Produktbildung führt. Die Kreise stehen für diese Variante FAΔ34 (siehe auch Fig. 2).

### Beispiel 4: Einfluß der Lage der Transskriptionsterminatorsequenz.

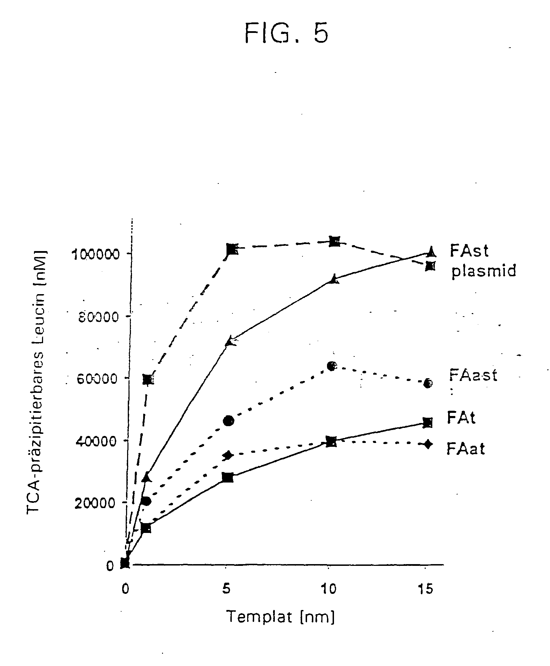

Zur Untersuchung des Einflusses der Lage der Terminatorsequenz wurden die Produkte FAst und FAast (siehe Fig. 2) erzeugt. Beide sind identisch mit FAt und FAat, außer daß eine 22 bp Spacersequenz zwischen dem Stopcodon und dem Terminator mittels unterschiedlicher Primer eingeführt wurde. In der Fig. 5 erkennt man, daß die Spacersequenz eine etwa 2-fache Erhöhung der Expression bewirkt.

In der Figur 5 ist aber auch aus einem Vergleich von FAt und FAat weiterhin zu erkennen, daß ein Affinitytag kaum Einfluß auf die Expression hat.

### Beispiel 5: PCR aus einer komplexen DNA Mischung.

Die Effektivität und Spezifität des erfindungsgemäßen Verfahrens wurde in Gegenwart einer hohen Menge an kompetitiver DNA untersucht. Eine PCR für FAst wurde entsprechend der vorstehenden Beschreibungen ausgeführt mit den folgenden Ausnahmen: die Nukleinsäurebasissequenz wurde in Konzentrationen von 0,16 bis 20 pg/50 µl Reaktorvolumen durchgeführt und die Reaktionen wurden mit 0,83 µg chromosomaler DNA aus Escherichia coli, ultraschallbehandelt für 5 min., supplementiert. Es wurde gefunden, daß weder die Qualität noch die Quantität des PCR Produkts durch die Anwesenheit eines 5-millionenfachen Überschusses an kompetitiver DNA beeinflußt wurde.

### Beispiel 6: Affinitätsreinigung mit Strep-tag II.

Eine Reaktionsmischung mit 10 µg des radioaktiv markierten FAast wurde der Affinitätsreinigung unterworfen. Etwa 81 % des aufgetragenen Materials wurde von der Säule erhalten und 67 % konnten als reines Produkt in den Elutionsfraktionen gewonnen werden (berechnet aus TCA Präzitipation der Fraktionen der Affinitätssäule).

### Beispiel 7: Aktivität des PCR Produkts.

Proben von H-FABP, synthetisiert entweder mittels des Plasmids oder als PCR Produkt FAast, wurden miteinander hinsichtlich der Bindungsaktivität für Oleinsäure untersucht. Nach der Transskription/Translation wurden verschiedene Volumina mit 0 bis 330 pmol nicht-markierten H-FABP in einem Bindungsassay gemäß der vorstehenden Beschreibung zu Methoden untersucht. Die Aktivitäten wurden als identisch gefunden; unabhängig von dem Herstellungsweg.

### Beispiel 8: Stabilität des PCR Produkts.

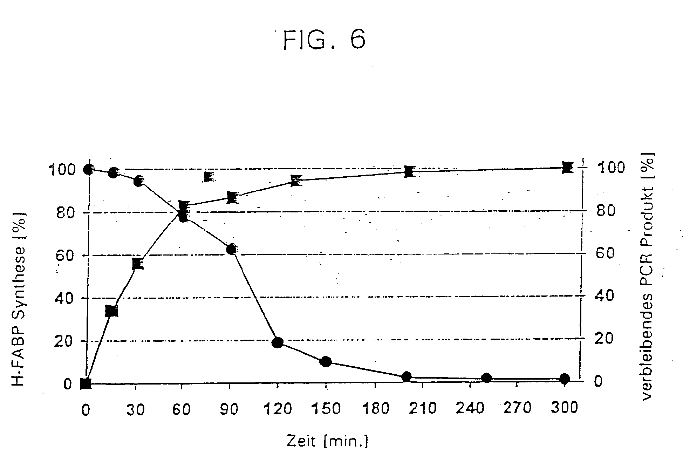

Zur Untersuchung, ob die Stabilität des PCR Produkts möglicherweise die Effektivität der Expression begrenzen könnte, wurde die Abnahme des PCR Produktes FAast gemessen. Es wurde hierfür das radioaktiv markierte Produkt eingesetzt. In bestimmten Zeitabständen wurden Aliquoten des Reaktionsgemisches entnommen und mittels denaturierender Polyacrylamid Gelelektrophorese untersucht. Die Menge am verbleibendem PCR Produkt wurde durch Scannen der Radioaktivität des Gels quantifiziert und mit dem Zeitverlauf der Proteinsynthese, gemessen durch Scannen der Radioaktivität von H-FABP im Gel nach Auftrennung der Reaktionsmischungen mittels SDS-PAGE, verglichen. Die Ergebnisse sind in der Figur 6 dargestellt. Man erkennt, daß die Halbwertzeit des PCR Produkts ca. 100 min. beträgt, was dem Einlauf der H-FABP Synthese in ein Plateau entspricht.

### Beispiel 9: Optimierte Bedingungen für eine PCR mit vier Primern.

In der Tabelle I sind optimierte Bedingungen für eine PCR mit vier Primern in einem Reaktionsvolumen von 25 µl zusammengefaßt.

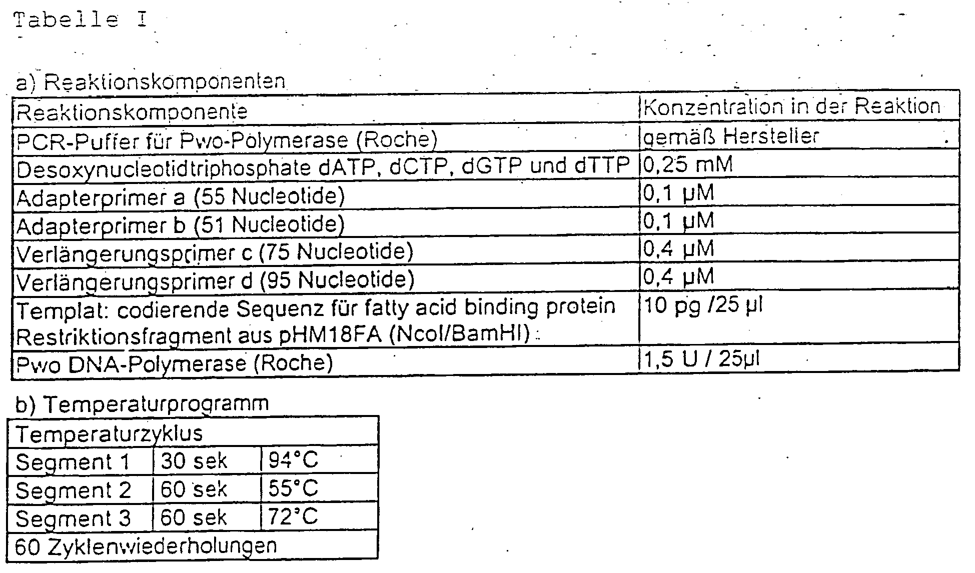

### Beispiel 10: PCR mit sechs Primern.

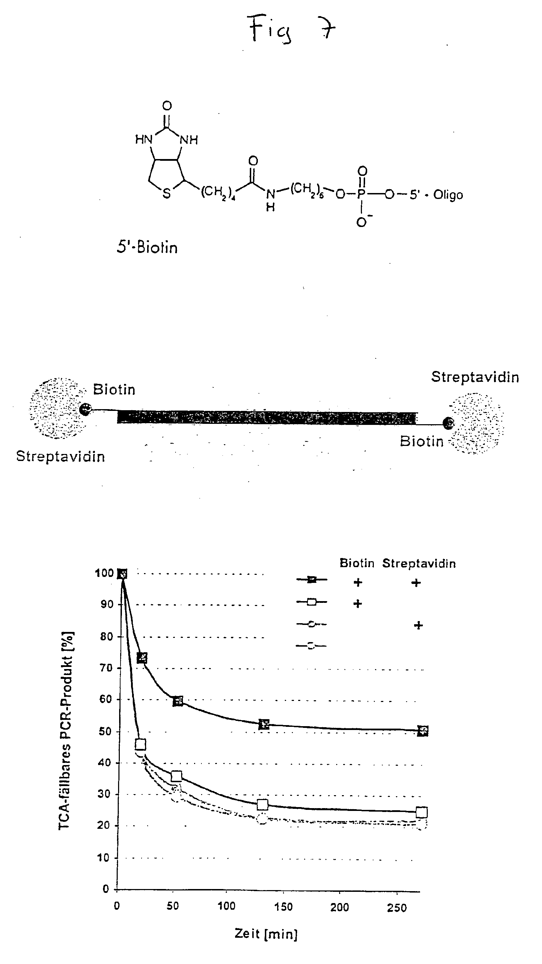

Mit den Materialien aus Beispiel 9, jedoch mit zusätzlich zwei Amplifikationsprimern e (26 Nucleotide) und f (33 Nucleotide) sowie einer erhöhten Adapterprimer-Konzentration von 0,2 µM wurden variierende Verlängerungsprimer Konzentrationen eingestellt. Bezüglich der Amplifikationsprimer wird auf die Figur 1 verwiesen, dort BIOR und BIOF. BIOF ist ein biotinmarkierter Forwardprimer und BIOR ein biotinmarkierter Reverseprimer. Die Struktur ist in der Fig. 7 dargestellt.

Ein minimaler Bedarf an teurem Verlängerungsprimer ergab sich, wenn zunächst 25 Zyklen ohne Amplifikationsprimer und anschließend weitere 25 Zyklen mit Amplifikationsprimer gefahren wurden. Die Konzentration an Verlängerungsprimer konnte durch den Einsatz der Amplifikationsprimer bis auf 0,025 µM gesenkt werden, ein Faktor von ca. 1/20, bei dennoch verbesserter Homogenität und Ausbeute an PCR Produkt.

Diese Vorteile beruhen darauf, daß die Wahrscheinlichkeit der Bildung von Zwischenprodukten in hohen Konzentrationen mit dem Einsatz der sechs Primer stark reduziert ist, da die Primer, die zur Entstehung der Zwischenprodukte erforderlich sind, in geringen Konzentrationen eingesetzt werden. Zwischenprodukte können folglich nicht mit den Amplifikationsprimern exponentiell angereichert werden.

### Beispiel 11: PCR mit sechs Primern in zwei Stufen.

Grundsätzlich wird mit den Materialien, wie vorstehend angegeben, gearbeitet. Zunächst wird eine Vor-PCR in einem Reaktionsvolumen von 5 µl mit 0,1 pg Nukleinsäurebasissequenz durchgeführt, und zwar mit 0,3 µM Adapterprimer und 0,5 µM Verlängerungsprimer über 20 Zyklen. Dann wird die so erhaltene Reaktionslösung mit PCR Ansatzvolumen auf 25 µl verdünnt. Dann wird Amplifikationsprimer auf eine Endkonzentration von 0,5 µM zugegeben. Schließlich wird für weitere 30 Zyklen amplifiziert.

### Beispiel 12: Stabilisierung einer Nukleinsäure mit Biotin.

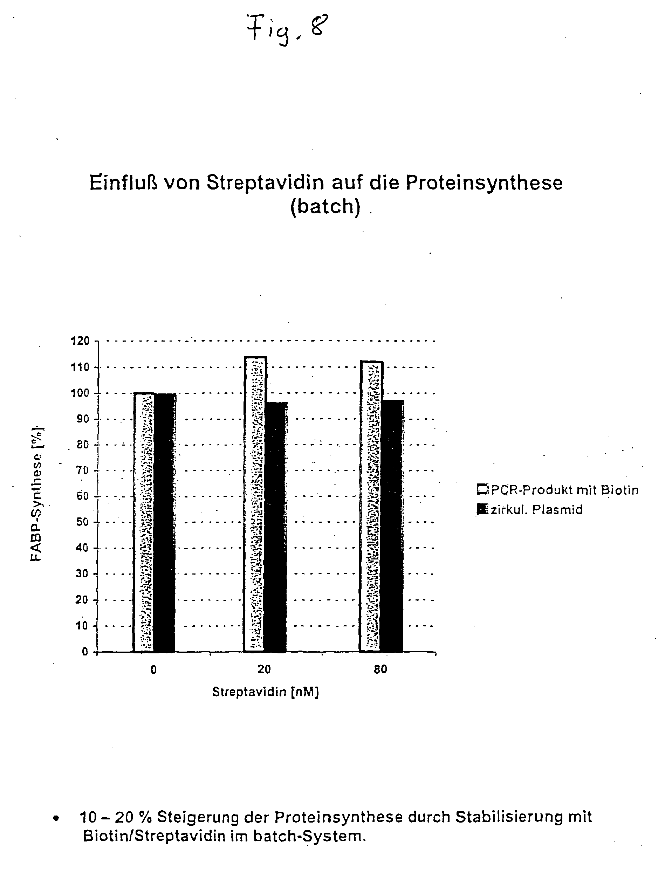

Es wurde unter Einsatz der Primer BIOF und BIOR im Wege der PCR mit 6 Primern, wie vorstehend beschrieben, eine Nukleinsäure hergestellt und deren Abbau als Funktion der Zeit sowie die Verbesserung der Proteinsynthese untersucht. Dies ist in den Figuren 7 und 8 dargestellt. Man erkennt, daß sich mit Biotin, insbesondere nach Umsatz mit Streptavidin eine erheblich verbesserte Stabilität ergibt. Dies führt auch zu einer bis zu 20% höheren Proteinsynthese.

Unabhängig von den vorstehenden Beispielen ist anzumerken, daß mit dem erfindungsgemäßen Verfahren auch sehr leicht Variationen der Sequenzen durch Mutationen möglich sind, beispielsweise durch Einsetzen von Taq-Polymerase und/oder veränderten Reaktionsbedingungen. Wenn dies nicht gewünscht ist, so kann vorzugsweise mit Pwo oder Pfu gearbeitet werden, welche genauer funktionieren und proofreading Aktivität haben.

## Patentansprüche

1. Verfahren zur präparativen Herstellung von langen Nukleinsäuren mittels PCR und mit den folgenden Hybridisierungsschritten:
a) eine Nukleinsäurenbasissequenz wird am 3' und 5' Ende, bezogen auf doppelsträngige Nukleinsäuren, wobei die Hybridisierung jeweils am 5'-Ende des sense- und antisense-Stranges erfolgt, mit jeweils einem Adapterprimer A und B, welche jeweils eine mit den jeweiligen Enden der Nukleinsäurebasissequenz hybridisierbare Teilsequenz aufweisen, hybridisiert,
b) das Produkt aus Stufe a) wird am 3' und 5' Ende, nämlich den außenliegenden freien Enden der Adapterprimer A und B, mit jeweils einem Verlängerungsprimer C und D, welcher eine Verlängerungssequenz enthält und welche jeweils eine mit den jeweiligen Enden der Adapterprimer A und B hybridisierbare Teilsequenz aufweisen, hybridisiert,
wobei aus der Nukleinsäurebasissequenz eine an dem 3' und dem 5' Ende der Nukleinsäurebasissequenz um die Verlängerungssequenzen verlängerte und amplifizierte Nukleinsäuresequenz gebildet wird.

2. Verfahren nach Anspruch 1, wobei das Produkt aus der Stufe b) in einer Stufe c) am 3' und 5' Ende mit jeweils einem Amplifikationsprimer hybridisiert wird, wobei eine amplifizierte Nukleinsäureendsequenz gebildet wird.

3. Verfahren nach Anspruch 1 oder 2, wobei die Adapterprimer < 70 Nucleotide enthalten, wobei die Verlängerungsprimer ≥ 70 Nucleotide enthalten, und/oder wobei die Amplifikationsprimer < 70 Nukleotide enthalten.

4. Verfahren nach einem der Ansprüche 1 bis 3, wobei die Schritte a), b) und optional der Schritt c) in einer PCR Lösung enthaltend die Nukleinsäurebasissequenz, die Adapterprimer A und B, die Verlängerungsprimer C und D und optional die Amplifikationsprimer durchgeführt werden.

5. Verfahren nach einem der Ansprüche 1 bis 4, wobei die Schritte a) und b) in einer Verfahrenstufe A) in einer Vor-PCR Lösung enthaltend die Nukleinsäurebasissequenz, die Adapterprimer A und B und die Verlängerungsprimer C und D für eine definierte erste Zyklenzahl durchgeführt werden und wobei der Schritt c) in einer Verfahrenstufe B) in einer Haupt-PCR Lösung enthaltend das PCR Produkt aus der Stufe A) und die Amplifikationsprimer für eine definierte zweite Zyklenzahl durchgeführt wird.

6. Verfahren nach einem der Ansprüche 1 bis 4, wobei die PCR in einem Reaktionsvolumen von 10 bis 100 µl, vorzugsweise 20 bis 40 µl, mit 0,01 bis 100 pg, vorzugsweise 1 bis 50 pg, Nukleinsäurebasissequenz, 0,05 bis 10 µM, vorzugsweise 0,1 bis 5 µM, Adapterprimer und 0,005 bis 0,5 µM, vorzugsweise 0,001 bis 0,1 µM, Verlängerungsprimer durchgeführt wird, wobei nach einer definierten Anfangszyklenzahl 0,01 bis 10 µM, vorzugsweise 0,1 bis 5 µM Amplifikationsprimer zugegeben werden, und wobei mittels einer definierte Anzahl anschließender Zyklen die amplifizierte Nukleinsäureendsequenz hergestellt wird.

7. Verfahren nach Anspruch 5, mit den folgenden Reaktionsbedingungen:
Stufe A): Reaktionsvolumen < 10 µl; 0,001 bis 5 pg, vorzugsweise 0,01 bis 1 pg, Nukleinsäurebasissequenz; 0,05 bis 10 µM, vorzugsweise 0,1 bis 5 µM, Adapterprimer und 0,05 bis 10 µM, vorzugsweise 0,1 bis 5 µM, Verlängerungsprimer; erste Zyklenzahl 10 bis 30, vorzugsweise 15 bis 25,
Stufe B): Reaktionsvolumen 10 bis 100 µl, vorzugsweise 15 bis 50 µl, erhalten durch Ergänzung der Lösung aus Stufe A) mit PCR-Ansatzlösung; 0,01 bis 10 µM, vorzugsweise 0,1 bis 5 µM, Amplifikationsprimer; zweite Zyklenzahl 15 bis 50, vorzugsweise 20 bis 40.

8. Verwendung eines Verfahrens nach einem der Ansprüche 1 bis 7 zur Herstellung von Nukleinsäuren für die zellfreie in vitro Proteinbiosynthese, insbesondere in prokaryontischen Systemen, oder für in vitro Transkriptionssysteme.

9. Verwendung nach Anspruch 8 in einem Translationsystem aus Escheria coli D10.

10. Verwendung eines Verfahrens nach einem der Ansprüche 1 bis 7 zur selektiven Amplifikation einer definierten Nukleinsäurebasissequenz aus einer Nukleinsäurebibliothek.

11. Verwendung eines Verfahrens nach einem der Ansprüche 1 bis 7 zur Charakterisierung von Gensequenzen, wobei die Gensequenz als Nukleinsäurenbasissequenz eingesetzt wird und wobei das erhaltene Protein hinsichtlich Struktur und/oder Funktion analysiert wird.

## Claims

1. A method for preparative production of long nucleic acids by PCR and involving the following hybridization steps:
a) a nucleic acid base sequence is hybridized on the 3' and 5' ends, related to doublestranded nucleic acids, wherein the hybridization takes place respectively at the 5' end of the sense and antisense strand, with an adapter primer A and B, respectively, comprising a partial sequence hybridizing with the respective ends of the nucleic acid base sequence,
b) the product from step a) is hybridized on the 3' and 5' ends, namely at the outside free ends of the adapter primers A and B, with one extension primer C and D each containing an extension sequence, and comprising a partial sequence hybridizing with the respective ends of the adapter primers A and B,
wherein a nucleic acid sequence enlarged by the extension sequences and amplified on the 3' and 5' ends of the nucleic acid base sequence is formed from the nucleic acid base sequence.

2. A method according to claim 1, wherein the product from step b) is hybridized in a step c) on the 3' and 5' ends with one amplification primer each, an amplified nucleic acid end sequence being formed.

3. A method according to claim 1 or 2, wherein the adapter primers contain < 70 nucleotides, wherein the extension primers contain ≥ 70 nucleotides, and/or wherein the amplification primers contain < 70 nucleotides.

4. A method according to one of claims 1 to 3, wherein the steps a), b) and as an option the step c) are performed in a PCR solution containing the nucleic acid base sequence, the adapter primers A and B, the extension primers C and D and as an option the amplification primers.

5. A method according to one of claims 1 to 4, wherein the steps a) and b) in a method step A) are performed in a pre-PCR solution containing the nucleic acid base sequence, the adapter primers A and B and the extension primers C and D for a defined first number of cycles, and wherein the step c) in a method step B) is performed in a main PCR solution containing the PCR product from the step A) and the amplification primers for a defined second number of cycles.

6. A method according to one of claims 1 to 4, wherein the PCR is performed in a reaction volume of 10 to 100 µl, preferably 20 to 40 µl with 0.01 to 100 pg, preferably 1 to 50 pg nucleic acid base sequence, 0.05 to 10 µM, preferably 0.1 to 5 µM adapter primer, and 0.005 to 0.5 µM, preferably 0.001 to 0.1 µM extension primer, wherein after a defined number of starting cycles 0.01 to 10 µM, preferably 0.1 to 5 µM amplification primer are added, and wherein by means of a defined number of subsequent cycles the amplified nucleic acid end sequence is produced.

7. A method according to claim 5, comprising the following reaction conditions:
Step A): reaction volume < 10 µl; 0.001 to 5 pg, preferably 0.01 to 1 pg nucleic acid base sequence; 0.05 to 10 µM, preferably 0.1 to 5 µM adapter primer, and 0.05 to 10 µM, preferably 0.1 to 5 µM extension primer; first number of cycles 10 to 30, preferably 15 to 25,
Step B): reaction volume 10 to 100 µl, preferably 15 to 50 µl, obtained by complementing the solution from step A) with PCR starting solution; 0.01 to 10 µM, preferably 0.1 to 5 µM amplification primer; second number of cycles 15 to 50, preferably 20 to 40.

8. The use of a method according to one of claims 1 to 7 for the production of nucleic acids for the cell-free in vitro protein biosynthesis, in particular in prokaryotic systems, or for in vitro transcription systems.

9. The use according to claim 8 in a translation system of Escherichia coli D10.

10. The use of a method according to one of claims 1 to 7 for the selective amplification of a defined nucleic acid base sequence from a nucleic acid library.

11. The use of a method according to one of claims 1 to 7 for the characterization of gene sequences, wherein the gene sequence is used as a nucleic acid base sequence and wherein the obtained protein is analyzed with regard to structure and/or function.

## Revendications

1. Procédé pour la production préparative d'acides nucléiques longs au moyen de RPC et comprenant les étapes d'hybridation suivantes:
a) une séquence de base d'un acide nucléique est hybridée aux extrémités 3' et 5', par rapport aux acides nucléiques à double brins, dans laquelle l'hybridation se passe respectivement à l'extrémité 5' du brin sens et antisens, avec une amorce d'adaptation A et B, respectivement, comprenant une séquence partielle chacune hybridant avec les extrémités respectives de la séquence de base d'un acide nucléique,
b) le produit à partir de l'étape a) est hybridé aux extrémités 3' et 5', c'est-à-dire aux extrémités extérieures libres des amorces d'adaptation A et B, avec une amorce d'extension C et D chacune comprenant une séquence d'extension, et comprenant une séquence partielle chacune hybridant avec les extrémités respectives des amorces d'adaptation A et B,
dans lequel à partir de la séquence de base d'un acide nucléique, une séquence d'un acide nucléique élargie par les séquences d'extension et amplifiée aux extrémités 3' et 5' de la séquence de base d'un acide nucléique est formée.

2. Procédé selon la revendication 1, dans lequel le produit à partir de l'étape b) est hybridé dans une étape c) aux extrémités 3' et 5' avec une amorce d'amplification chacune, une séquence finale d'un acide nucléique amplifiée étant formée.

3. Procédé selon la revendication 1 ou 2, dans lequel les amorces d'adaptation comprennent < 70 nucléotides, dans lequel les amorces d'extension comprennent ≥ 70 nucléotides, et/ou dans lequel les amorces d'amplification comprennent < 70 nucléotides.

4. Procédé selon une des revendications 1 à 3,
dans lequel les étapes a), b) et comme option l'étape c) sont exécutées dans une solution RPC comprenant la séquence de base d'un acide nucléique, les amorces d'adaptation A et B, les amorces d'extension C et D et comme option les amorces d'amplification.

5. Procédé selon une des revendications 1 à 4,
dans lequel les étapes a) et b) sont exécutées dans une étape du procédé A) dans une solution RPC préliminaire comprenant la séquence de base d'un acide nucléique, les amorces d'adaptation A et B et les amorces d'extension C et D pour un premier nombre défini de cycles, et dans lequel l'étape c) est exécutée dans une étape du procédé B) dans une solution RPC principale comprenant le produit RPC à partir de l'étape A) et les amorces d'amplification pour un deuxième nombre défini de cycles.

6. Procédé selon une des revendications 1 à 4,
dans lequel la RPC est exécutée dans un volume de réaction de 10 à 100 µl, de préférence 20 à 40 µl avec 0,01 à 100 pg, de préférence 1 à 50 pg de séquence de base d'un acide nucléique, 0,05 à 10 µM, de préférence 0,1 à 5 µM d'amorce d'adaptation, et 0,005 à 0,5 µM, de préférence 0,001 à 0,1 µM d'amorce d'extension, dans lequel après un nombre de départ défini de cycles 0,01 à 10 µM, de préférence 0,1 à 5 µM d'amorce d'amplification sont ajoutés, et dans lequel au moyen d'un nombre défini de cycles subséquents la séquence finale d'un acide nucléique amplifiée est produite.

7. Procédé selon la revendication 5, comprenant les conditions de réaction suivantes:
Étape A): volume de réaction < 10 µl; 0,001 à 5 pg, de préférence 0,01 à 1 pg de séquence de base d'un acide nucléique; 0,05 à 10 µM, de préférence 0,1 à 5 µM d'amorce d'adaptation, et 0,05 à 10 µM, de préférence 0,1 à 5 µM d'amorce d'extension; premier nombre de cycles 10 à 30, de préférence 15 à 25,
Étape B): volume de réaction 10 à 100 µl, de préférence 15 à 50 µl, obtenu par complément de la solution à partir de l'étape A) avec la solution RPC de départ; 0,01 à 10 µM, de préférence 0,1 à 5 µM d'amorce d'amplification; deuxième nombre de cycles 15 à 50, de préférence 20 à 40.

8. Utilisation d'un procédé selon une des revendications 1 à 7 pour la production d'acides nucléiques pour la biosynthèse acellulaire in vitro, en particulier dans des systèmes procaryotes ou pour des systèmes de transcription in vitro.

9. Utilisation selon la revendication 8 dans un système de translation à partir d'Escherichia coli D10.

10. Utilisation d'un procédé selon une des revendications 1 à 7 pour l'amplification sélective d'une séquence de base définie d'un acide nucléique à partir d'une banque d'acides nucléiques.

11. Utilisation d'un procédé selon une des revendications 1 à 7 pour la caractérisation de séquences de gène, dans laquelle la séquence de gène est utilisée comme séquence de base d'un acide nucléique et dans laquelle la protéine obtenue est analysée par rapport à la structure et/ou la fonction.
